# EUROPEAN PATENT SPECIFICATION

(11) **EP 1 955 667 B1**
(45) Date of publication and mention of the grant of the patent: **01.12.2010**
(21) Application number: 08250433.3
(22) Date of filing: 06.02.2008
(51) Int. Cl.: A61B 17/88, B25B 23/10, A61B 17/00, A61B 17/86, B25B 15/00

(54) **Global nail screw retaining screwdriver**
Globaler nagelschraubenhaltender Schraubendreher
Tournevis global de rétention de vis et de clous

(30) Priority: 08.02.2007 US 888840 P
(43) Date of publication of application: 13.08.2008
(73) Proprietor: Zimmer Technology, Inc., Warsaw, IN 46580 (US)
(72) Inventor: Parrott, Rebecca L., Winona Lake, Indiana 46590 (US); Pacelli, Nicolas J., Culver, Indiana 46511 (US); Bodle, Kerry, South Bend, Indiana 46644 (US)
(74) Representative: Mays, Julie

(56) References cited:
- WO-A-2004/089245
- US-A- 2 763 304
- US-A- 4 936 172
- US-A1- 2005 216 027

## Description

### BACKGROUND

### 1. Field of the Invention.

The present invention relates to screwdrivers, and, more particularly, to a screwdriver having an apparatus for selectively retaining a screw thereon.

### 2. Description of the Related Art.

It is known to use screw-type devices and associated drivers for bone fracture repair. It can be helpful to selectively couple such screw-type devices to the driver. US 2, 763, 304 describes a screwdriver adapted to couple a cross-head screw. US 2005/0216027 A1 relates to a bone fastener which can engage a bone screw.

Expense associated with surgical procedures is often influenced by the time necessary to perform the surgery, and therefore by the time needed in a surgical operating room. Screw-type devices used for bone fracture repair are ultimately deposited and left within bone. Whereas affixing screws to their drivers provides assurance of the relative position of the screw and driver, an increased degree of screw to driver fixation is often associated with an increased time to effect screw and driver disassociation. Accordingly, increased surety of screw to driver fixation is often achieved at the expense of an expedient later-desired screw and driver disassociation.

### SUMMARY

The present disclosure generally describes a screwdriver and a mating screw that selectively couple and uncouple at the direction of a user.

The present disclosure provides, in combination, a bone fastener and a fastener drive tool according to claim 1. The drive tool including a handle, a shaft coupled to the handle and including a bit portion on a distal end thereof for engaging a socket of a fastener, and a lock coupled to the shaft for selectively coupling a fastener thereto, the lock having a first lock position to couple the drive tool to the fastener and a second lock position allowing the drive tool to be separated from the fastener. The bone fastener including a socket having at least one shoulder defined therein such that the lock engages the shoulder when the lock is in the first lock position.

The present disclosure also provides a fastener tool. The fastener tool includes a handle, a shaft coupled to the handle and inducing a bit portion on a distal end thereof for engaging a socket of a fastener, and a lock means for selectively coupling a fastener to the shaft. The lock has a first lock position to couple the drive tool to the fastener and a second lock position allowing the drive tool to be separable from the fastener.

It is described a method of attaching a fastener to a patient. The method includes the steps of providing a fastener tool having a lock on a distal end thereof and a switch capable of positioning the lock, placing the lock in a first position, placing the distal end of the tool intro a drive socket of the fastener, engaging the lock to couple the fastener to the fastener tool, affixing the fastener within the anatomy of a patient, disengaging the lock to disengage the fastener from the fastener tool, and removing the distal end of the tool from the drive socket of the fastener.

### BRIEF DESCRIPTION OF THE DRAWINGS

The above-mentioned and other features of this invention, and the manner of attaining them, will become more apparent and the invention itself will be better understood by preference to the following description of an embodiment of the invention taken in conjunction with the accompanying drawings, wherein:

Figure 1 is a perspective view of a driver and associated screw of the present disclosure;

Figures 2A&B are partial exploded views of a handle end of the driver of Figure 1;

Figure 3A is a perspective view of the driver of Figure 1 in a disengaged orientation;

Figure 3B is a partially cross-sectioned view of a head end of the driver and screw of Figure 3A;

Figure 3C is a perspective view of the head end of the driver of Figure 3A;

Figure 3D is an end view of the screw of Figure 1 with an internal void shown in phantom;

Figure 4A is a perspective view of the driver of Figure 1 in an engaged orientation;

Figure 4B is a partially cross-sectioned view of a head end of the driver and screw of Figure 4A;

Figure 4C is a perspective view of the head end of the driver of Figure 4A;

Figure 4D is an overhead view of the screw of Figure 1 with an internal void shown in phantom and the head end of the driver of Figure 4A.

Corresponding reference characters indicate corresponding parts throughout the several views. The exemplification set out herein illustrates one preferred embodiment of the invention, in one form, and such exemplification is not to be construed as limiting the scope of the invention in any manner.

### DETAILED DESCRIPTION OF THE INVENTION

The description that follows refers to a retrograde femoral nail application. While described with respect to a retrograde femoral nail application, the principles of the present invention can be applied to other surgical and non-surgical applications.

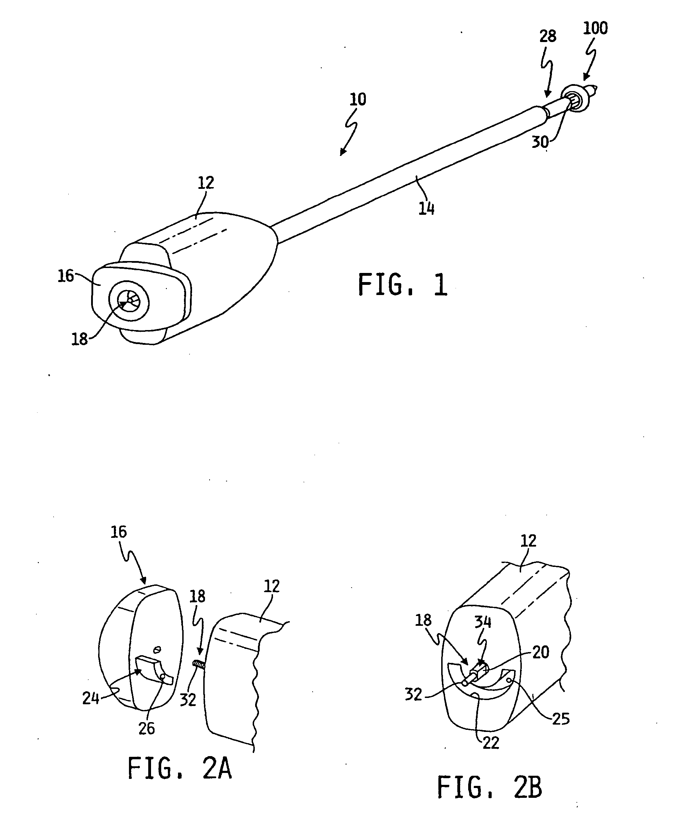

Figure 1 shows screwdriver 10 and associated bone screw 100. Screwdriver 10 includes handle 12, shaft 14, positive lock 16, locking member 18, and a tension/disassembly knob (not shown). Fig. 2B shows handle 12 with positive lock 16 removed therefrom. Handle 12 includes passageway 20 therein that aligns with a similarly diametered passageway (not shown) within shaft 14. Handle 12 further includes a proximal end having partially-annular slot 22 defined therein.

Screwdriver 10 is configured to securely and selectively couple to bone screw 100. By operating positive lock 16, a user may couple and uncouple screw 100 from a distal end of screwdriver 100. Accordingly, a compact design to prevent unintended disassociation is provided.

Slot 22 of handle 12 is sized to receive a tab or lug 24 defined on positive lock 16 therein. Slot 22 includes two recesses 25 (only one shown) at the bottom thereof sized to receive a detent 26 defined on tab 24 of positive lock 16. The arc length of slot 22 defines the permitted rotational travel of tab 24 and therefore the permitted travel of positive lock 16 relative to handle 12. The length of slot 22 and locations of recesses 25 combine to define the pre-set rotational positions of locking member 18 permitted by screwdriver 10. Positive lock 16 acts as a switch between the pre-set rotational positions. Screwdrivers 10 having a hex driving pattern utilize pre-set rotational positions offset by about 30 degrees from each other. Screwdrivers 10 having a square driving pattern (not shown) utilize pre-set rotational positions offset by about 45 degrees from each other. Screwdrivers 10 having a triangular driving pattern (not shown) utilize pre-set rotational positions offset by about 60 degrees from each other. More generally, for any given driving pattern, pre-set rotational positions are defined by the relationship of: rotational offset angle = 180/(number of sides of the selected driving pattern).

When moving from one recess 25 to the other, detent 26 provides tactile feedback and an audible click once detent 26 is seated within a recess 25. Other embodiments provide a recess in positive lock 16 also. In such embodiments, a ball (not shown) is located within recess 25 of positive lock 16. The click can be facilitated by the use of a silicon O-ring (not shown) that compresses until the final position is achieved and the ball seats in a recess of slot 22 of handle 12.

The exterior of handle 12 is sized and shaped to easily and comfortably fit within the hand of a user. A distal end of handle 12 is coupled to shaft 14. It should be appreciated that when the pieces are discussed, "distal" and "proximal" are used relative to the user of screwdriver 10 such that the distal end of screwdriver 10 is farther from the user than the proximal end of screwdriver 10. One embodiment provides a recess (not shown) at the distal end of handle 12 that has an interior cross-section shaped to receive shaft 14 so as to prevent rotation therebetween. Exemplary shapes of the cross section of the recess include hexagonal, square, triangular, and chorded off circles. Shaft 14 may be permanently fixed within the recess, may provide a friction fit within the recess, and may be readily removable via use of the tension/disassembly knob and discussed below.

Shaft 14 includes a proximal end sized and shaped to be received within the distal end of handle 12 as previously discussed. Shaft 14 further includes a distal end 28 that provides a bit portion having driving surfaces 30. Distal end 28 is hexagonal in the provided embodiment. Square, triangle, and other non-circular drivers are also specifically envisioned. Shaft 14 also includes a passageway (not shown) sized to receive screw locking member 18 partially therein.

Screw locking member 18 includes, from proximal to distal, an assembly attachment 32, a chorded positive lock engagement portion 34, a cylindrical portion or rod (not shown), and a lock portion 38. Assembly attachment 32 passes through positive lock 16 and provides for attachment of screw locking member 18 to the tension/disassembly knob. Such attachment may be via a threaded engagement, a void and cotter pin, or any other suitable connection. Chorded positive lock engagement portion 34 is received in a mating bore in positive lock 16. The engagement between lock engagement portion 34 and positive lock 16 prevents relative rotation therebetween such that rotation imparted to positive lock 16 is translated to locking member 18. The cylindrical portion (not shown) of screw locking member 18 has a substantially fixed diameter and extends through handle 12 and shaft 14. Lock portion 38 is coupled to the distal end of the cylindrical portion (not shown) and is of a cross section geometry substantially identical to the cross section geometry of distal end 28 of shaft 14. Other embodiments are envisioned where the cross section geometry of lock portion 38 is substantially different than then cross section geometry of distal end 28 of shaft 14. One such embodiment includes a cross-shaped lock portion 38 on a hex-shaped distal end 28 of shaft 14. Yet another alternative for lock portion 38 provides one or more cams positioned to exert tension between screwdriver 10 and screw 100. Lock portion 38 may assume most any shape such that a cross sectional perimeter of lock portion 38 is within or equal to a perimeter of distal end 28 when lock portion 38 is in one of the pre-set positions and the cross-sectional perimeter of lock portion 38 extends beyond the perimeter of distal end 28 when in a second of the pre-set positions.

Screwdriver 10 is provided to work with traditional hex (or whatever driver is chosen) screws. Additionally, screws 100 are provided to work with screwdriver 10 and to be selectively retained thereon.

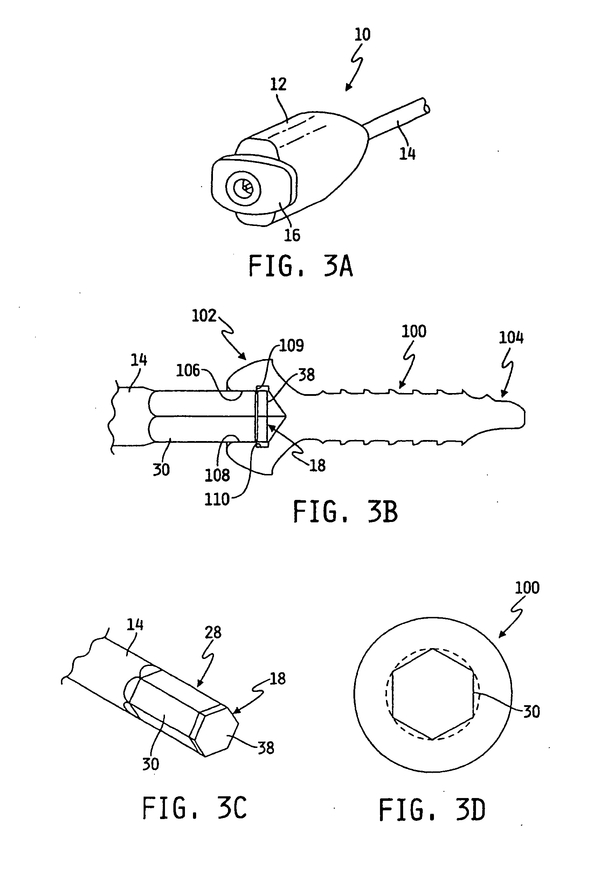

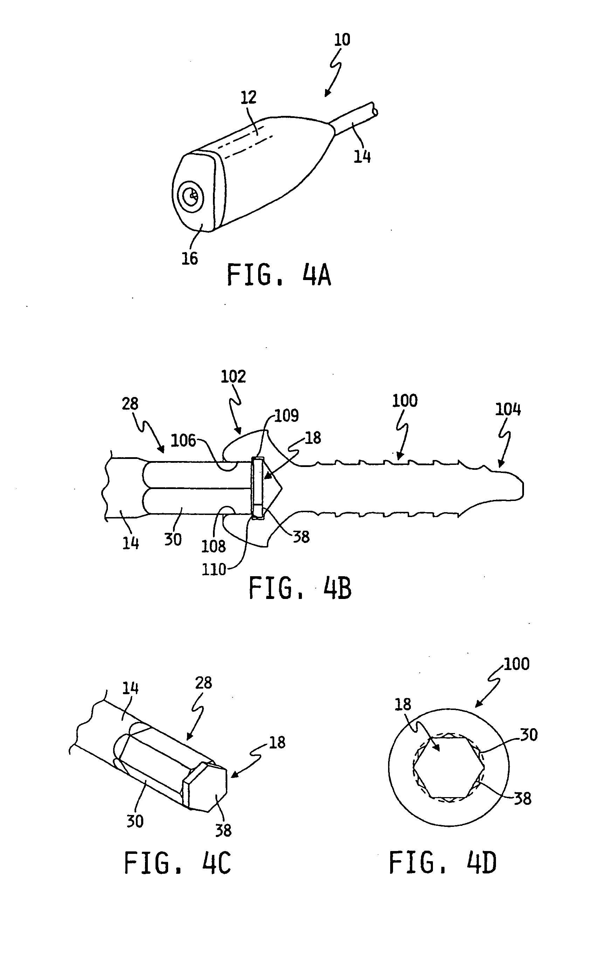

Screws 100 each include a head 102 and a threaded shaft 104. Threaded shaft 104 may be of any design of various pitches and may be made from various materials as appropriate for the selected application. As shown in Figs. 3B and 4B, head 102 includes a socket/bore 106 having a driving section 108 and a lock section 110. Driving section 108 is shaped to engage the selected driver (hex, Phillips, square, triangle, etc.). Driving section 108 receives distal end 28 of shaft 14 and lock portion 38 of locking member 18 to allow rotation to be imparted to screw 100 from screwdriver 10. Lock section 110 is substantially circular groove and, in one embodiment, is positioned deeper within screw 100 than driving section 108. Lock section 110 is of a substantially constant diameter equal to the "opposite corner-to-corner" distance of drive section 108 so as to allow lock portion 38 to selectively rotate therein. Accordingly, a plurality of shoulders 109 are created at the interface between lock section 110 and driving section 108

In use, positive lock 16 is rotated to a first position to be misaligned with handle 12 as shown in Fig. 3A and so that lock portion 38 is aligned with distal end 28 of shaft 14, as shown in Fig. 3C. Lock portion 38 and distal end 28 of shaft 14 are then inserted into bore 106 such that lock portion 38 is positioned at a depth to align with lock section 110 as shown in Fig. 3B. Once so positioned, positive lock 16 is rotated to a second position to be aligned with handle 12 as shown in Fig. 4A. This causes lock portion 38 to be misaligned with distal end 28 of shaft 14, as shown in Fig. 4C. This misalignment results in lock portion 38 traveling rotationally within lock section 110 of screw 100 and causes lock portion 38 to engage shoulders 109 to achieve a lock position, as shown in Figs. 4B and D. Additionally, detent 26 seats within recess 25 to frictionally hold the relative positions of lock portion 38 and drive surfaces 30. Screwdriver 10 and screw 100 are thereby directly coupled.

During a surgery, such as for securing a femoral nail application, screws 100 are placed to secure a nail in the intermedullary canal. Whereas the placement of the nail often utilizes an open procedure, performing the placement of screw 100 often involves soft tissue that must be traversed by screw 100 in order for screw 100 to arrive at the point where it will be affixed.

Screws 100 are often provided via a screw caddy (not shown). The locking of lock portion 38 and lock section 110 allows screws to be retrieved from the caddy directly by screwdriver 10.

Once screw 100 traverses the extra-osseous tissue, the engagement of driving surfaces 30 with driving section 108 of screw 100 allows rotational force to be applied to screw 100 via screwdriver 10 to effect the desired placement of screw 100. Additionally, coupling screw 100 to screwdriver 10 via lock portion 38 allows the interconnection without increase in torque being applied to screw 100. While screwdriver 10 and screw are within the anatomy of a patient, positive lock 16 provides a visual and tactile indication of the lock portion 38 orientation.

Once so placed, positive lock 16 is rotated relative to handle 12 back to the first position, shown in Fig. 3A. This rotation releases screwdriver 10 from screw 100. Screwdriver 10 is then retracted from the anatomy of the patient.

Additionally, placement of screws 100 often utilizes a cannula (not shown). The interconnection of lock portion 38 and lock section 110 allows selective fixation without increasing the diameter of the screwdriver 10 and screw 100 combination. Accordingly, the combination is compatible with typical existing cannulas. Furthermore, the combination is compatible with typically used incision sizes.

## Claims

1. A combination of a bone fastener (100) and a fastener tool (10), the fastener tool (10) including,
a handle (12);
a shaft (14) coupled to the handle (12) and including a bit portion on a distal end (28) thereof for engaging a socket (106) of a fastener (100); and
a lock means (18) for selectively coupling the fastener (100) to the shaft (14), the lock means (18) having a first lock position to couple the fastener tool (10) to the fastener (100) and a second lock position allowing the fastener tool (10) to be separable from the fastener (100), wherein the bit portion defines a cross sectional perimeter and the lock means (18) defines a perimeter that extends beyond the perimeter of the bit portion when the lock means (18) is in the first lock position and that is within or equal to the perimeter of the bit portion when the lock means (18) is in the second lock position,
the bone fastener (100) including:
a socket (106) having at least one shoulder (109) defined therein such that the lock means (18) engages the shoulder (109) when the lock means (18) is in the first lock position, **characterised in that** the socket (106) includes a substantially annular groove (110).

2. The combination of claim 1, wherein the fastener tool (10) further includes a switch (16) having a first switch position and a second switch position, the first switch position causing the lock means (18) to assume the first lock position and the second switch position causing the lock means (18) to assume the second lock position, the switch (16) moving from the first switch position to the second switch position by rotation.

3. The combination of claim 2, wherein the switch (16) provides a physical indication to a user that indicates the position of the lock means (18).

4. The combination of claim 2, wherein the switch (16) is coupled to a proximal end of the handle (12).

5. The combination of claim 1, wherein the lock means (18) is positioned distally relative to the bit portion.

6. The combination of claim 1, wherein the lock means (18) has a substantially similar cross section as a lock portion of the socket (106) of the fastener (100).

7. The combination of claim 1, wherein the lock means (18) has a non-circular cross-section.

8. The combination of claim 1, wherein the lock means (18) includes a rod disposed within a cannula of the shaft (14).

9. The combination of claim 1, wherein the lock means (18) includes a lock portion disposed (38) on the bit portion of the shaft (14), the lock portion (38) having a substantially similar cross section as the bit portion.

10. The combination of claim 1, wherein the lock means (18) is rotatably coupled to the shaft (14).

11. The combination of claim 1, further including a switch (16) having a first switch position and a second switch position, the first switch position causing the lock means (18) to assume the first lock position and the second switch position causing the lock means (18) to assume the second lock position and the lock means (18) is rotationally fixed relative to the switch (16).

## Patentansprüche

1. Kombination aus einem Knochenfixierelement (100) und einem Fixierwerkzeug (10), wobei das Fixierwerkzeug (10) aufweist:
einen Griff (12);
einen Schaft (14), der mit dem Griff (12) verbunden ist und an seinem distalen Ende (28) einen Endabschnitt für den Eingriff mit einem Einsatz (106) eines Fixierelements (100) aufweist; und
eine Verriegelungseinrichtung (18) zum selektiven Verbinden des Fixierelements (100) mit dem Schaft (14), wobei die Verriegelungseinrichtung (18) eine erste Verriegelungsstellung zum Verbinden des Fixierwerkzeugs (10) mit dem Fixierelement (100) und eine zweite Verriegelungsstellung aufweist, die eine Trennung des Fixierwerkzeugs (10) von dem Fixierelement (100) zuläßt, wobei der Endabschnitt eine äußere Querschnittsbegrenzung definiert und die Verriegelungseinrichtung (18) eine äußere Begrenzung definiert, die sich über die äußere Begrenzung des Endabschnitts hinaus erstreckt, wenn sich die Verriegelungseinrichtung (18) in der ersten Verriegelungsstellung befindet, und die innerhalb oder auf der äußeren Begrenzung des Endabschnitts liegt, wenn sich die Verriegelungseinrichtung (18) in der zweiten Verriegelungsstellung befindet,
wobei das Knochenfixierelement (100) aufweist:
einen Einsatz (106) mit mindestens einer Schulter (109), die darin so bestimmt ist, daß die Verriegelungseinrichtung (18) mit der Schulter (109) in Eingriff kommt, wenn sich die Verriegelungseinrichtung (18) in der ersten Verriegelungsstellung befindet, **dadurch gekennzeichnet, daß** der Einsatz (106) eine im wesentlichen ringförmige Nut (100) aufweist.

2. Kombination nach Anspruch 1, wobei das Fixierwerkzeug (10) ferner einen Schalter (16) mit einer ersten Schaltstellung und einer zweiten Schaltstellung aufweist, wobei die erste Schaltstellung bewirkt, daß die Verriegelungseinrichtung (18) die erste Verriegelungsstellung annimmt, und die zweite Schaltstellung bewirkt, daß die Verriegelungseinrichtung (18) die zweite Verriegelungsstellung annimmt, wobei sich der Schalter (16) durch Drehung aus der ersten Schaltstellung in die zweite Schaltstellung bewegt.

3. Kombination nach Anspruch 2, wobei der Schalter (16) eine physikalische Anzeige für einen Benutzer liefert, welche die Stellung der Verriegelungseinrichtung (18) anzeigt.

4. Kombination nach Anspruch 2, wobei der Schalter (16) mit einem proximalen Ende des Griffs (12) verbunden ist.

5. Kombination nach Anspruch 1, wobei die Verriegelungseinrichtung (18) distal zum Endabschnitt angeordnet ist.

6. Kombination nach Anspruch 1, wobei die Verriegelungseinrichtung (18) einen im wesentlichen ähnlichen Querschnitt wie ein Verriegelungsabschnitt des Einsatzes (106) des Fixierelements (100) aufweist.

7. Kombination nach Anspruch 1, wobei die Verriegelungseinrichtung (18) einen nicht kreisförmigen Querschnitt aufweist.

8. Kombination nach Anspruch 1, wobei die Verriegelungseinrichtung (18) einen Stab aufweist, der innerhalb einer Kanüle des Schafts (14) angeordnet ist.

9. Kombination nach Anspruch 1, wobei die Verriegelungseinrichtung (18) einen Verriegelungsabschnitt (38) aufweist, der am Endabschnitt des Schafts (14) angeordnet ist, wobei der Verriegelungsabschnitt (38) einen im wesentlichen ähnlichen Querschnitt wie der Endabschnitt aufweist.

10. Kombination nach Anspruch 1, wobei die Verriegelungseinrichtung (18) drehbar mit dem Schaft (14) gekoppelt ist.

11. Kombination nach Anspruch 1, die ferner einen Schalter (16) mit einer ersten Schaltstellung und einer zweiten Schaltstellung aufweist, wobei die erste Schaltstellung bewirkt, daß die Verriegelungseinrichtung (18) die erste Verriegelungsstellung annimmt, und die zweite Schaltstellung bewirkt, daß die Verriegelungseinrichtung (18) die zweite Verriegelungsstellung einnimmt und die Verriegelungseinrichtung (18) bezüglich des Schalters (16) drehfixiert wird.

## Revendications

1. Combinaison d'un élément (100) de fixation osseuse et d'un outil (10) à élément de fixation, l'outil (10) à élément de fixation comportant,
un manche (12) ;
un arbre (14) couplé au manche (12) et comportant une partie d'embout sur une extrémité distale (28) de celui-ci pour engager une douille (106) d'un élément de fixation (100) ; et
un moyen de verrouillage (18) pour coupler de manière sélective l'élément de fixation (100) à l'arbre (14), le moyen de verrouillage (18) ayant une première position de verrouillage pour coupler l'outil (10) à élément de fixation à l'élément de fixation (100) et une deuxième position de verrouillage permettant à l'outil (10) à élément de fixation d'être séparé de l'élément de fixation (100), où la partie d'embout définit un périmètre en coupe transversale et le moyen de verrouillage (18) définit un périmètre qui s'étend au-delà du périmètre de la partie d'embout lorsque le moyen de verrouillage (18) se trouve dans la première position de verrouillage et qui est dans la plage du ou égal au périmètre de la partie d'embout lorsque le moyen de verrouillage (18) se trouve dans la deuxième position de verrouillage,
l'élément (100) de fixation osseuse comportant :
une douille (106) ayant au moins un épaulement (109) qui y est défini de sorte que le moyen de verrouillage (18) se mette en prise avec l'épaulement (109) lorsque le moyen de verrouillage (18) se trouve dans la première position de verrouillage, **caractérisée en ce que** la douille (106) comporte une rainure (110) essentiellement annulaire.

2. Combinaison de la revendication 1, dans laquelle l'outil (10) à élément de fixation comporte en outre un commutateur (16) ayant une première position de commutation et une deuxième position de commutation, la première position de commutation amenant le moyen de verrouillage (18) à adopter la deuxième position de verrouillage et la deuxième position de commutation amenant le moyen de verrouillage (18) à adopter la deuxième position de verrouillage, le commutateur (16) se déplaçant de la première position de commutation à la deuxième position de commutation par rotation.

3. Combinaison de la revendication 2, dans laquelle le commutateur (16) fournit une indication physique à un utilisateur qui indique la position du moyen de verrouillage (18).

4. Combinaison de la revendication 2, dans laquelle le commutateur (16) est couplé à une extrémité proximale du manche (12).

5. Combinaison de la revendication 1, dans laquelle le moyen de verrouillage (18) est positionné de manière distale par rapport à la partie d'embout.

6. Combinaison de la revendication 1, dans laquelle le moyen de verrouillage (18) a une section transversale essentiellement similaire qu'une partie de verrouillage de la douille (106) de l'élément de fixation (100).

7. Combinaison de la revendication 1, dans laquelle le moyen de verrouillage (18) a une section transversale non-circulaire.

8. Combinaison de la revendication 1, dans laquelle le moyen de verrouillage (18) comporte une tige disposée dans une canule de l'arbre (14).

9. Combinaison de la revendication 1, dans laquelle le moyen de verrouillage (18) comporte une partie de verrouillage (38) disposée sur la partie d'embout de l'arbre (14), la partie de verrouillage (38) ayant une section transversale essentiellement similaire que la partie d'embout.

10. Combinaison de la revendication 1, dans laquelle le moyen de verrouillage (18) est couplé en rotation à l'arbre (14).

11. Combinaison de la revendication 1, comportant en outre un commutateur (16) ayant une première position de commutation et une deuxième position commutateur, la première position de commutation amenant le moyen de verrouillage (18) à adopter la première position de verrouillage et la deuxième position de commutation amenant le moyen de verrouillage (18) à adopter la deuxième position de verrouillage et le moyen de verrouillage (18) est fixé de manière rotative par rapport au commutateur (16).
